# EUROPÄISCHE PATENTSCHRIFT

(11) **EP 4 410 252 B1**
(45) Veröffentlichungstag und Bekanntmachung des Hinweises auf die Patenterteilung: **20.08.2025**
(21) Anmeldenummer: 24154426.1
(22) Anmeldetag: 29.01.2024
(51) Int. Cl.: A61F 7/00

(54) **KÄLTEBEHANDLUNGSANORDNUNG**
REFRIGERATION TREATMENT ASSEMBLY
DISPOSITIF DE TRAITEMENT À FROID

(30) Priorität: 03.02.2023 DE 102023102667
(43) Veröffentlichungstag der Anmeldung: 07.08.2024
(73) Patentinhaber: crio ice GmbH & Co. KG, 75217 Birkenfeld (DE)
(72) Erfinder: SCHWEITZER, Reinhard, 72517 Birkenfeld (DE); KIRSCHENMANN, Günter, 76327 Pfinztal (DE)
(74) Vertreter: Zenz Patentanwälte Partnerschaft mbB

(56) Entgegenhaltungen:
- DE-A1- 102006 055 269
- DE-A1- 3 624 822
- KR-A- 20200 069 816
- US-A- 2 093 834
- US-A1- 2017 007 443

## Beschreibung

Die Erfindung betrifft eine Kältebehandlungsanordnung, aufweisend eine Kältekammer, welche eine sich in eine Vertikalrichtung erstreckende und einen Innenraum umschließende Umfangswandung mit einem Kopf-Ende und einem Boden-Ende aufweist, einen Einströmkanal, welcher wenigstens eine in den Innenraum weisende Austrittsöffnung aufweist, einen Verdampferkanal, welcher mit dem Einströmkanal strömungsverbunden ist und in welchem ein ein Kältemittel bevorratendes Kältemittel-Behältnis zur Erzeugung eines Kaltluft-Gemisches entnehmbar eingesetzt ist, und eine mit dem Verdampferkanal verbundene und zum Ansaugen von Luft aus der Umgebung ausgebildete Fördervorrichtung, welche ferner zum Fördern eines Kaltluft-Gemisches von dem Verdampferkanal zu der wenigstens einen in den Innenraum weisenden Austrittsöffnung ausgebildet ist, wobei es sich bei dem Kaltluft-Gemisch um ein Gemisch aus in dem Verdampferkanal verdampftem Kältemittel und der aus der Umgebung angesaugten Luft handelt, und wobei die Fördervorrichtung außerhalb der Kältekammer angeordnet ist.

Eine Kältebehandlungsanordnung der vorstehend genannten Art ist zum Beispiel aus der DE 10 2006 055269 A1 bekannt.

Aus der US 2017/007443 A1 ist ein kryogenes Zuführungs- und Kontrollsystem bekannt, welches von einem externen Kryogen-Vorratsbehälter gespeist wird. Der Kryogen-Vorratsbehälter ist mit einem Nassraum verbunden, der in enger Verbindung mit einem von Menschen besetzen Trockenraum oder einer Kammer steht. Die besetzen Kammer wird für eine kryogenbasierte, eisige Atmosphäre in der Größenordnung von etwa -100 °C bis etwa -160 °C zur Kühlung und Behandlung lebender Körper verwendet.

Ferner ist aus der KR 2020 0069816 A ein Kryotherapiesystem bekannt, welches eine Kammer zur Aufnahme einer Person und zur Zufuhr eines Gases für die Kryotherapie, einen Auslassabschnitt zum Auslassen des Gases in Richtung des Inneren der Kammer, ein Verbindungsrohr, das einen Durchgang definiert, durch den das Gas zugeführt wird und durch den sich das Gas zum Auslassabschnitt bewegen kann, einen Einstellabschnitt, der an einem Durchgang des Gases angeordnet ist, um zumindest den Druck des Gases einzustellen, und einen ersten Steuerabschnitt, der mit dem Einstellabschnitt verbunden ist, um den Einstellabschnitt zu steuern, aufweist.

Aus der DE 36 24 822 A1 ist eine Vorrichtung zur Durchführung einer Kyrotherapie am ganzen Körper mit einem kalten Behandlungsgas bekannt, wobei die Vorrichtung aus einem Behandlungsraum aus isolierendem Material zur Aufnahme des Patienten besteht. Der Behandlungsrau besitzt Anschlüsse zur Zufuhr des Behandlungsgases und ist als offene Halbschale ausgebildet, in deren hinterem Teil Öffnungen zur Ableitung des Behandlungsgases und in deren seitlichen Teilen Öffnungen zur Zufuhr des Behandlungsgases in das Innere der Halbschale angeordnet sind.

Schließlich ist aus der US 2 093 834 A ein Beatmungsmittel mit einer aufblasbaren Abdeckung für den menschlichen Körper bekannt. Die Abdeckung umfasst mindestens drei Lagenabschnitte aus einem flexiblen Material, die zur Ausbildung einer Vielzahl von Kammern zusammengehalten werden, und Mittel zum kontinuierlichen Leiten von Druckluft in eine der Kammern zwischen zwei der Lagenabschnitte, um die Abdeckung aufgeblasen und in Kontakt mit dem Körper zu halten. Einer der Lagenabschnitte ist porös ausgebildet, um ein kontinuierliches Entweichen von Luft aus der Kammer zu ermöglichen.

Unter dem Begriff Kryotherapie hat sich in den letzten Jahrzehnten eine Methode der extremen Kälteanwendung etabliert, die als Ganzkörperkältetherapie unter sehr tiefen Temperaturen in einer Kältekammer bezeichnet wird und bei welcher der Körper gezielt tiefen Temperaturen ausgesetzt wird. Dies soll die Stoffwechselvorgänge auf Zellebene positiv beeinflussen. Diese Art der Therapie wurde anfänglich bei der Behandlung von Rheumapatienten eingesetzt und findet verstärkt Einzug in die Sport- und Schmerztherapie. Die Ganzkörperkältetherapie kann ferner auch bei psychosomatischen Erkrankungen, im Bereich von Fitness und Beauty sowie zur allgemeinen Steigerung des körperlichen und mentalen Wohlbefindens eingesetzt werden.

Bei den Kältekammern kann die Kältebehandlung in einem einzigen Raum (eine sogenannte 1-Zellen-Kältekammer) oder auch stufenweise mehrstufig (sogenannte 2-Zellen- oder 3-Zellen-Kältekammern) erfolgen. Bei der mehrstufigen Kältebehandlung wird die zu behandelnde Person für wenige Minuten in einem äußeren Behandlungsraum bei einer ersten kalten Temperatur, häufig im Bereich von -50°C bis -70°C, auf die eigentliche Kryotherapie bei unter -100°C vorbereitet. Zudem wird die zu behandelnde Person im äußeren Behandlungsraum auch von Feuchtigkeit getrocknet, um Verletzungen durch Erfrierungen und zusätzliches Unbehagen durch die Feuchtigkeit auf der Haut zu vermeiden. Anschließend betritt die zu behandelnde Person den inneren Behandlungsraum, wo die eigentliche Therapie bei einer Temperatur kleiner -100°C erfolgt.

Die Kältebereitstellung erfolgt bei aus dem Stand der Technik bekannten Kältekammern durch unterschiedliche Techniken.

Bei einem Großteil der am Markt verfügbaren Kältekammern wird durch Kaskadenkälteanlagen mit fluorierten Kältemitteln gekühlt. Die der Kältekammer zugeführte Luft wird dabei mehrstufig über mehrere Verdampfer abgekühlt, wobei nicht umweltfreundliche Kältemittel eingesetzt werden. Diese bekannten Kältekammern sind zum Teil wartungsintensiv und erfordern durch die anlagenbedingt hohen Drücke zusätzliche Sicherheitsmaßnahmen.

Alternativ ist es bekannt, dass zur Kältebereitstellung flüssiger Stickstoff in Verbindung mit einem oder mehreren Verdampfern, in denen der Stickstoff verdampft, eingesetzt wird. Die Verdampfer sind jeweils in einem Luftstrom, welcher der Kältekammer zugeführt wird, angeordnet, wobei die Luft sich beim Überströmen der Verdampfer durch Wärmeleitung abkühlt. Allerdings sind die Bereitstellung und Lagerung des flüssigen Stickstoffs einerseits kostenintensiv und andererseits ist der Einsatz gesamtenergetisch ineffizient.

Somit weisen die bekannten Kältekammern und deren Kälteerzeugung den Nachteil auf, dass sie sowohl in der Anschaffung als auch im Betrieb sehr hohe Kosten verursachen.

Aufgabe der Erfindung ist es daher, eine Kältebehandlungsanordnung bereitzustellen, welche einen besonders energie- und kosteneffizienten Betrieb ermöglicht.

Diese Aufgabe wird erfindungsgemäß durch eine Kältebehandlungsanordnung mit den Merkmalen gemäß dem Anspruch 1 gelöst.

Die erfindungsgemäße Kältebehandlungsanordnung stellt eine Kältedusche dar und umfasst eine Kältekammer, welche eine sich in eine Vertikalrichtung erstreckende und einen Innenraum umschließende Umfangswandung mit einem Kopf-Ende und einem Boden-Ende aufweist, einen Einströmkanal, welcher wenigstens eine in den Innenraum weisende Austrittsöffnung aufweist, einen Verdampferkanal, welcher mit dem Einströmkanal strömungsverbunden ist und in welchem ein ein Kältemittel bevorratendes Kältemittel-Behältnis zur Erzeugung eines Kaltluft-Gemisches entnehmbar eingesetzt ist, und eine mit dem Verdampferkanal verbundene und zum Ansaugen von Luft aus der Umgebung ausgebildete Fördervorrichtung, welche ferner zum Fördern eines Kaltluft-Gemisches von dem Verdampferkanal zu der wenigstens einen in den Innenraum weisenden Austrittsöffnung ausgebildet ist, wobei es sich bei dem Kaltluft-Gemisch um ein Gemisch aus in dem Verdampferkanal verdampftem Kältemittel und der aus der Umgebung angesaugten Luft handelt, und wobei die Fördervorrichtung außerhalb der Kältekammer angeordnet ist. Erfindungsgemäß erfolgt die Wärmeübertragung von dem Kältemittel auf die angesaugte Luft mittels Konvektion. Erfindungsgemäß ist der Einströmkanal an dem Kopf-Ende der Umfangswandung angeordnet, von wo aus das Kaltluft-Gemisch in die Kältekammer (2) einströmt und aufgrund seiner Dichte in Richtung des Boden-Endes (5) sinkt, wobei die Fördervorrichtung ferner zum Umströmen der aus der Umgebung angesaugten Luft über das Kältemittel derart ausgebildet ist, dass das Umströmen des Kältemittels mit der aus der Umgebung angesaugten Luft einen Wärmeaustausch mittels Konvektion zwischen der Luft und dem Kältemittel bewirkt und zur Verdampfung des Kältemittels führt.

Durch die Erfindung wird eine einzellige Kältebehandlungsanordnung, d.h. eine Kältebehandlungsanordnung mit einer 1-Zellen-Kältekammer, zur Verfügung gestellt, welche sich durch einen funktionsgerechten Aufbau auszeichnet und mit deren Hilfe kosteneffiziente Kältebehandlungen durchführbar sind. Zusätzlich ist die erfindungsgemäße Kältebehandlungsanordnung bzw. Kältedusche aufgrund ihres funktionsgerechten Aufbaus in ihrer Anschaffung sehr kostengünstig.

In Ausgestaltung der Erfindung ist vorgesehen, dass das Kältemittel für die Wärmeübertragung durch Konvektion aus Kohlensäurepellets besteht und das verdampfte Kältemittel ein Kohlenstoffdioxid-Dampf ist. Die Kältebehandlungsanordnung wird folglich mit Kohlensäurepellets, d.h. mit Trockeneis, betrieben, was handelsüblich einfach zu beziehen ist und eine sehr tiefe Temperatur innerhalb der Kältekammer erzeugt. Von besonderem Vorteil ist es bei diesem Kältemittel, dass es sublimiert, also verdampft, ohne dabei Rückstände oder Feuchtigkeit zu hinterlassen.

Für eine einfach zu handhabende Auffüllung des Kältemittel-Behältnisses sieht die Erfindung in weiterer Ausgestaltung vor, dass der Verdampferkanal eine verschließbare Öffnung aufweist, durch die hindurch das Kältemittel-Behältnis zur Befüllung mit Kältemittel aus dem Verdampferkanal entnehmbar und innerhalb des Verdampferkanals wieder einbringbar ist.

Zur Einstellung der Intensität der Behandlung mit dem Kaltluft-Gemisch sieht die Erfindung in weiterer Ausgestaltung vor, dass die Fördervorrichtung als ein steuerbares und/oder stufenlos regelbares Gebläse ausgebildet ist.

Für die Verdampfung des Kältemittels ist es konstruktiv besonders günstig, wenn das Kältemittel-Behältnis aus einem Edelstahldrahtgeflecht nach Art eines Korbes ausgebildet. Durch diese Ausgestaltung kann die angesaugte Luft auf einfache Weise auf das Kältemittel geleitet werden.

Ein kostengünstiger Aufbau der Kältebehandlungsanordnung lässt sich in Ausgestaltung der Erfindung ferner dadurch realisieren, dass die Kältekammer wenigstens an dem Kopf-Ende stirnseitig offen ausgebildet ist. Mit Hilfe eines solchen Aufbaus ragt der Kopf der zu behandelnden Person aus der Kältekammer heraus, so dass ein aufwendiger Schutz des Kopfes, wie er sonst bei Kältekammern notwendig ist, wenn sich die zu behandelnde Person vollständig innerhalb der Kältekammer befindet, nicht erforderlich ist.

Zur Anpassung an die Körpergröße der zu behandelnden Person sieht die Erfindung in weiterer Ausgestaltung vor, dass sich der Einströmkanal von dem Kopf-Ende in Richtung des Boden-Endes erstreckt und die wenigstens eine in den Innenraum weisende Austrittsöffnung in ihrer vertikalen Position einstellbar ausgebildet ist.

Zur vertikalen Einstellung bzw. zur Höheneinstellung besteht in weiterer Ausgestaltung der Erfindung eine konstruktiv günstige Möglichkeit darin, dass der Einströmkanal mit mehreren Austrittsöffnungen ausgebildet ist, welche hintereinanderliegend von dem Kopf-Ende in Richtung des Boden-Endes an dem Einströmkanal ausgebildet sind, wobei jeder Austrittsöffnung ein einstellbares Regulierungselement zugeordnet ist, welches zwischen einer die jeweilige Austrittsöffnung verschließenden Schließstellung und einer die jeweilige Austrittsöffnung freigebenden Öffnungsstellung bewegbar ausgebildet ist.

Ein einfaches Betreten und Verlassen der Kältekammer ist in weiterer Ausgestaltung der Erfindung dadurch möglich, dass die Umfangswandung mit wenigstens einem Wandelement und einem an dem wenigstens einen Wandelement schwenkbar gelagerten Türelement ausgebildet ist.

Kostengünstig ist es für die erfindungsgemäße Kältebehandlungsanordnung ferner, wenn die Umfangswandung aus EPP-Hartschaum bzw. aus expandiertem Polypropylen ausgebildet ist.

Die Erfindung sieht in weiterer Ausgestaltung vor, dass ein Kammerkanal außenseitig an der Umfangswandung entlang verlaufend angeordnet ist und den Verdampferkanal mit dem Einströmkanal verbindet. Der Kammerkanal bildet primär die Verbindung zu dem Einströmkanal, wobei der Kammerkanal aber auch die Möglichkeit bietet, ein Schneerohr zur optionalen Zusatzkühlung und/oder Ersatzkühlung im Fall, dass das Kältemittel im Kältemittel-Behältnis weitestgehend verdampft ist, innerhalb des Kammerkanals anzuordnen. Die Anordnung des Schneerohrs, welches von einer Kohlensäure-Flasche bzw. CO₂-Gasflasche versorgt wird, muss nicht zwingend in dem Kammerkanal erfolgen - das Schneerohr ist auf jeden Fall stromauf der wenigstens einen in den Innenraum weisenden Austrittsöffnung vorzusehen, so dass eine Anordnung des Schneerohres auch innerhalb des Verdampferkanals in Betracht kommt.

Zur Erhöhung der Flexibilität der Kältebehandlungsanordnung sieht die Erfindung in weiterer Ausgestaltung vor, dass die Fördervorrichtung und der Verdampferkanal außerhalb der Kältekammer an einem Aufbaurahmen angeordnet sind. Mit dieser Ausgestaltung ist die Kältebehandlungsanordnung modular aufgebaut, so dass unterschiedliche Varianten von Aufbaurahmen angeboten werden können, welche sich zum Beispiel durch unterschiedliche Fördervorrichtungen unterscheiden.

In weiterer Ausgestaltung der Erfindung ist vorgesehen, dass an dem Aufbaurahmen ein Sauerstoffkonzentrator mit Behandlungs-Applikationen, wie zum Beispiel Sauerstoffmaske und Sicherheitsschlauch etc., angeordnet ist. Der in die Kältebehandlungsanordnung integrierte Sauerstoffkonzentrator zur Erzeugung eines reinen Sauerstoffs aus der atmosphärischen Luft sorgt in Kombination mit der Kälteanwendung mittels der Kältekammer für eine maximale Sauerstoff-Aufnahme, welche wiederum das Allgemeinbefinden und die Fitness der zu behandelnden Person in vorteilhafter Weise enorm verbessert.

Damit die der Kältekammer zugeführte Kaltluft entweichen kann, ist ferner in Ausgestaltung der Erfindung vorgesehen, dass die Kältekammer im Bereich des Boden-Endes einen Durchlass aufweist.

Schließlich sieht die Erfindung eine nichttherapeutische Verwendung von Kohlensäurepellets als Kältemittel für die vorstehend beschriebene Kältebehandlungsanordnung bzw. Kältedusche vor.

Es versteht sich, dass die vorstehend genannten und nachstehenden noch zu erläuternden Merkmale nicht nur in der jeweils angegebenen Kombination, sondern auch in anderen Kombinationen oder in Alleinstellung verwendbar sind, ohne den Rahmen der vorliegenden Erfindung zu verlassen. Der Rahmen der Erfindung ist nur durch die Ansprüche definiert.

Weitere Einzelheiten, Merkmale und Vorteile des Gegenstandes der Erfindung ergeben sich aus der nachfolgenden Beschreibung im Zusammenhang mit der Zeichnung, in der beispielhafte bevorzugte Ausführungsbeispiele der Erfindung dargestellt sind.

In der Zeichnung zeigt:
Figur 1 eine perspektivische Darstellung einer erfindungsgemäßen Kältebehandlungsanordnung gemäß eines ersten Ausführungsbeispiels,
Figur 2 eine weitere perspektivische Darstellung der Kältebehandlungsvorrichtung aus Figur 1,
Figur 3 eine perspektivische Darstellung einer Abwandlung der erfindungsgemäßen Kältebehandlungsvorrichtung aus Figur 1 gemäß eines zweiten Ausführungsbeispiels,
Figur 4 eine Seitenansicht der Kältebehandlungsvorrichtung aus Figur 3,
Figur 5 eine perspektivische Darstellung auf einen Aufbaurahmen,
Figur 6 eine perspektivische Darstellung eines Kältemittel-Behältnisses,
Figur 7 eine alternative Ausgestaltung für ein Podest der Kältebehandlungsvorrichtung,
Figur 8 eine perspektivische Ansicht einer Kältebehandlungsvorrichtung gemäß eines dritten Ausführungsbeispiels,
Figur 9 eine perspektivische Ansicht der Kältebehandlungsvorrichtung aus Figur 8, wobei ein Teil einer Umfangswandung nicht gezeigt ist, und
Figur 10 eine perspektivische und zum Teil geschnittene Ansicht der Kältebehandlungsvorrichtung aus Figur 8.

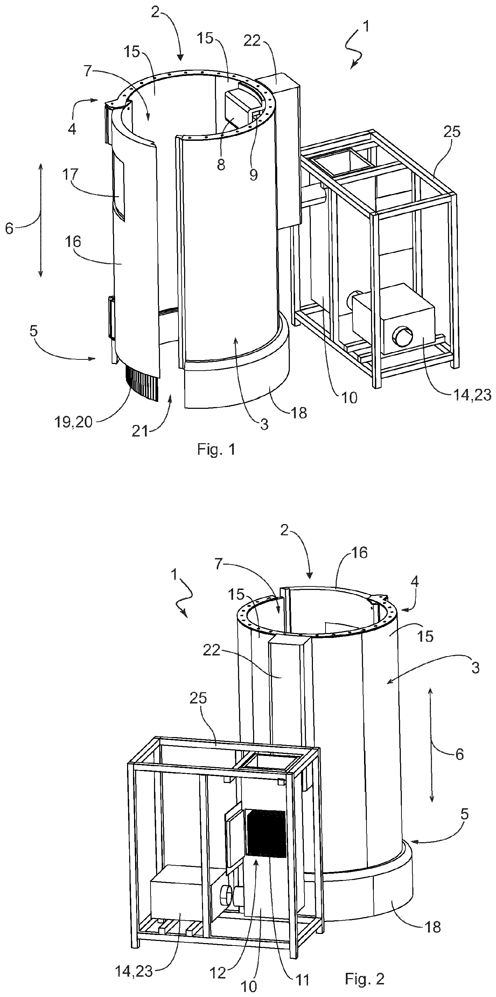

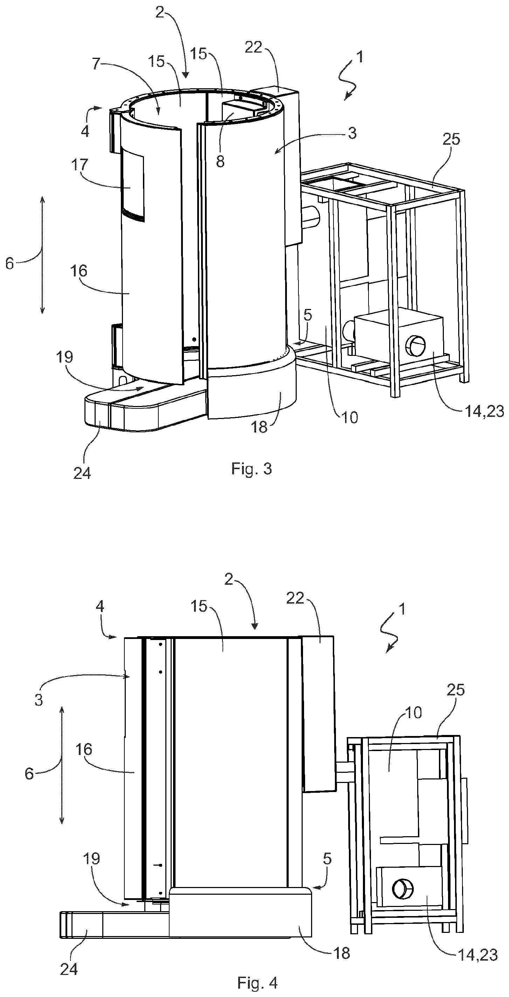

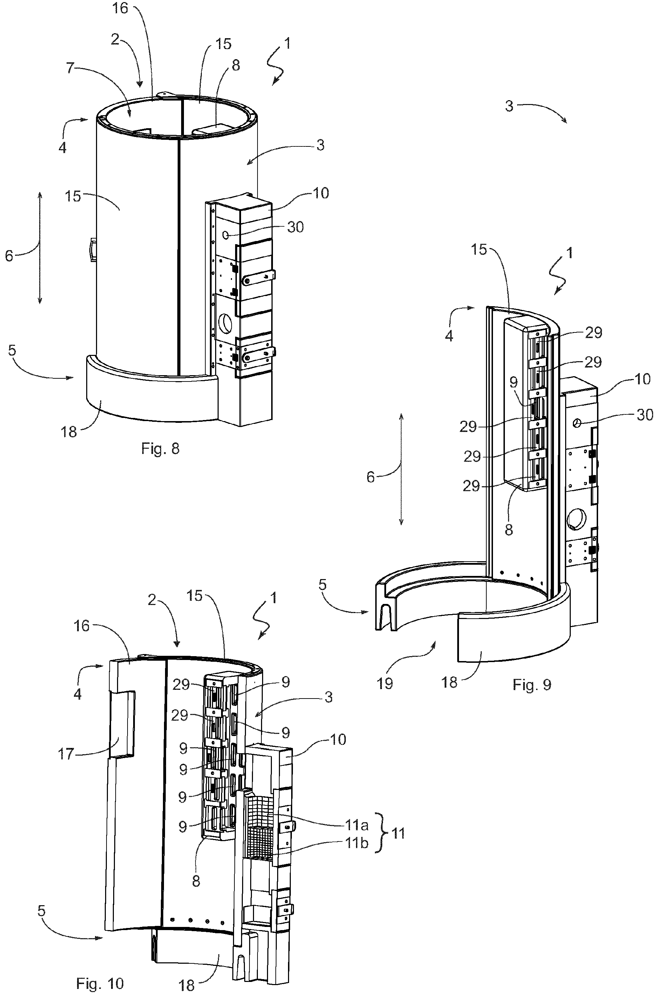

Die Figuren 1 bis 4 und 8 bis 10 zeigen jeweils in perspektivischen Darstellungen eine erfindungsgemäße Kältebehandlungsanordnung 1, wobei in den Figuren 1 und 2 ein erstes Ausführungsbeispiel, in den Figuren 3 und 4 ein zweites Ausführungsbeispiel und in den Figuren 8 bis 10 ein drittes Ausführungsbeispiel gezeigt ist. Alle Ausführungsbeispiele weisen grundsätzlich einen identischen Aufbau auf, so dass sich die nachstehende Beschreibung auf alle Ausführungsbeispiele und gleiche Bezugszeichen auf gleiche Bauteile oder Elemente mit gleicher Funktion beziehen. Auf die Unterschiede zwischen den beiden Ausführungsbeispielen wird explizit nachstehend eingegangen.

Die in den Figuren 1 bis 4 und 8 bis 10 gezeigte Kältebehandlungsanordnung 1 stellt eine Kältedusche dar und weist eine Kältekammer 2 mit einer Umfangswandung 3 auf. Die Umfangswandung 3, welche ein Kopf-Ende 4 und ein Boden-Ende 5 aufweist, erstreckt sich in eine Vertikalrichtung 6 und umschließt einen Innenraum 7. Die Größe des Innenraumes 7 und die Länge der Umfangswandung 3 in Vertikalrichtung 6 sind dabei derart ausgelegt, dass eine zu behandelnde Person mit ausreichend Abstand zu der Innenseite der Umfangswandung 3 innerhalb der Kältekammer 2 aufrecht stehen kann und der Kopf der zu behandelnden Person dabei aus dem Kopf-Ende 4 hervorsteht. Diesbezüglich ist bei allen Ausführungsbeispielen die Kältekammer 2 an ihrem Kopf-Ende 4 und damit stirnseitig offen ausgebildet. An dem Kopf-Ende 4 der Umfangswandung 3 ist ferner ein Einströmkanal 8 angeordnet, welcher wenigstens eine in den Innenraum 7 weisende Austrittsöffnung 9 aufweist. In den dargestellten Ausführungsbeispielen ist der Einströmkanal 8 innerhalb der Kältekammer 2 angeordnet. Über die Austrittsöffnung 9 des Einströmkanals 8 strömt zur Kältebehandlung der zu behandelnden Person ein Kaltluft-Gemisch in den Innenraum 7 ein.

Als Abwandlung zu dem ersten und zweiten Ausführungsbeispiel ist bei dem dritten Ausführungsbeispiel der Einströmkanal 8 derart ausgebildet, dass die Austrittsöffnung 9 in Vertikalrichtung 6 einstellbar anordenbar ist. Zu diesem Zweck erstreckt sich der Einströmkanal 8 von dem Kopf-Ende 4 in Richtung des Boden-Endes 5, wie aus den Figuren 8 bis 10 ersichtlich ist. Dabei weist der Einströmkanal 8 mehrere Austrittsöffnungen 9 auf. Die mehreren Austrittsöffnungen 9 sind jeweils schlitzförmig und paarweise auf sich gegenüberliegenden Seiten des Einströmkanals 8 ausgebildet. Wie beispielsweise aus Figur 10 hervorgeht, sind bei dem dritten Ausführungsbeispiel insgesamt fünf Paare von Austrittsöffnungen 9 vorgesehen, wobei auch eine davon abweichende Anzahl möglich ist. Die Austrittsöffnungen 9 sind hintereinanderliegend von dem Kopf-Ende 4 in Richtung des Boden-Endes 5 an dem Einströmkanal 8 ausgebildet. Dabei ist jeder Austrittsöffnung 9 ein einstellbares Regulierungselement 29 zugeordnet. Ein jeweiliges Regulierungselement 29 ist zwischen einer die jeweilige bzw. ihm zugeordnete Austrittsöffnung 9 verschließenden Schließstellung und einer die jeweilige bzw. ihm zugeordnete Austrittsöffnung 9 freigebenden Öffnungsstellung bewegbar ausgebildet. Ein jeweiliges Regulierungselement 29 ist bei dem gezeigten Ausführungsbeispiel in Form eines einstellbaren Schiebers ausgebildet. Wie zum Beispiel aus Figur 9 hervorgeht, ist das mittlere Regulierungselement 29 in der Öffnungsstellung angeordnet und gibt die zugeordnete Austrittsöffnung 9 für ein Ausströmen des Kaltluft-Gemisches frei, wohingegen die darüber und darunter liegend angeordneten Regulierungselemente 29 in ihrer Schließstellung angeordnet sind, so dass kein Kaltluft-Gemisch durch die zugeordneten Austrittsöffnungen 9 in den Innenraum 7 austreten kann. Zusätzlich zu der Höheneinstellung ist mit Hilfe der einstellbaren Regulierungselemente 29 ferner auch die Menge des in den Innenraum 7 zugeführten Kaltluft-Gemisches einstellbar, indem das Regulierungselement 29 neben der Schließstellung und der Öffnungsstellung auch in dazwischen liegenden Stellungen angeordnet werden kann.

Wie ferner beispielsweise die Figuren 1, 3, 9 und 10 zeigen, ist der Einströmkanal 8 mit einem Verdampferkanal 10 strömungsverbunden. In dem Verdampferkanal 10 ist ein Kältemittel-Behältnis 11 (siehe zum Beispiel Figuren 2 und 10) entnehmbar eingesetzt, wobei hierzu der Verdampferkanal 10 eine dichtend verschließbare und klappenartig ausgebildete Öffnung 12 aufweist, so dass das Kältemittel-Behältnis 11 zur Befüllung mit Kältemittel durch die Öffnung 12 hindurch aus dem Verdampferkanal 10 entnehmbar und in den Verdampferkanal 10 nach der Befüllung wieder einbringbar ist. Das Kältemittel-Behältnis 11 ist zur Bevorratung eines Kältemittels und zur Erzeugung eines Kaltluft-Gemisches ausgebildet, worauf nachstehend noch näher eingegangen wird.

Die Kältebehandlungsanordnung 1 weist ferner eine Fördervorrichtung 14 auf, welche lediglich für das erste und zweite Ausführungsbeispiel gezeigt ist, welche aber selbstverständlich auch bei dem dritten Ausführungsbeispiel vorgesehen ist. Die Fördervorrichtung 14 ist zum Ansaugen von Luft aus der Umgebung ausgebildet und mit dem Verdampferkanal 10 verbunden, so dass von der Fördervorrichtung 14 angesaugte Luft aus der Umgebung zu dem Verdampferkanal 10 gefördert wird. Dabei ist die Fördervorrichtung 14 derart ausgebildet, dass die aus der Umgebung angesaugte Luft über das in dem Verdampferkanal 10 angeordnete Kältemittel zur Wärmeübertragung mittels Konvektion gefördert wird. Die Fördervorrichtung 14 bläst somit die aus der Umgebung angesaugte Luft über das Kältemittel, so dass das Kältemittel von der aus der Umgebung angesaugten Luft umströmt wird. Wie den Figuren 1 bis 4 zu entnehmen ist, ist die Fördervorrichtung 14 außerhalb der Kältekammer 2 angeordnet. Die Fördervorrichtung 14 ist ferner zum Fördern des in dem Verdampferkanals 10 erzeugten Kaltluft-Gemisch von dem Verdampferkanal 10 zu der wenigstens einen in den Innenraum 7 weisenden Austrittsöffnung 9 ausgebildet. Bei dem Kaltluft-Gemisch handelt es sich erfindungsgemäß um ein Gemisch aus in dem Verdampferkanal 10 verdampftem Kältemittel und der aus der Umgebung angesaugten Luft, wobei das Umströmen des Kältemittels mit der aus der Umgebung angesaugten Luft einen Wärmeaustausch mittels Konvektion zwischen der Luft und dem Kältemittel bewirkt und zur Verdampfung des Kältemittels führt. Erfindungsgemäß besteht daher das Kältemittel aus Kohlensäurepellets bzw. Trockeneis mit einer Temperatur von ca. -80°C, wobei das verdampfte Kältemittel ein Kohlenstoffdioxid-Dampf ist. Zur verstellbaren Einstellung der Intensität des in den Innenraum 7 geförderten Kaltluft-Gemisches ist die Fördervorrichtung 14 als ein steuerbares und/oder stufenlos regelbares Gebläse 23 ausgebildet.

Wie die Figuren 1 bis 4 und 8 bis 10 zusätzlich zeigen, ist die Umfangswandung 3 mit Wandelementen 15 und einem an einem Wandelement 15 schwenkbar gelagerten Türelement 16 ausgebildet. Die Umfangswandung 3 und damit die Wandelemente 15 und das Türelement 16 sind aus EPP-Hartschaum ausgebildet. Dabei ist in dem Türelement 16 ein Sichtfenster 17 aus Acrylglas eingesetzt, was zum Beispiel eine Prüfung von außerhalb der Kältekammer 2 erlaubt, ob die Kältekammer 2 im Hinblick auf eine Kältebehandlung bereits ausreichend mit dem Kaltluft-Gemisch gefüllt ist. Das Boden-Ende 5 der Kältekammer 2 kann als Standfläche für die Kältekammer 2 ausgebildet sein. In dem in den Figuren gezeigten Ausführungsbeispiel ist hingegen an dem Boden-Ende 5 der Kältekammer 2 ein Sockel 18 angeordnet, welcher das Boden-Ende 5 der Kältekammer 2 abstützt und als eine Art Standfuß dient.

Bei den in den Figuren 1 bis 4 gezeigten Ausführungsbeispielen ist ein Kammerkanal 22 vorgesehen, welcher außenseitig an der Umfangswandung 3 entlang verlaufend angeordnet ist und den Verdampferkanal 10 mit dem Einströmkanal 8 verbindet. Ein Kammerkanal ist bei dem dritten Ausführungsbeispiel nicht vorgesehen, denn der Verdampferkanal 10 auf der Umfangswandung 3 angebracht ist. Innerhalb des Kammerkanals 22 kann für das erste und zweite Ausführungsbeispiel optional ein Schneerohr zur Zusatzkühlung angeordnet werden, wobei die Anordnung des Schneerohrs in jedem Fall stromauf der Austrittsöffnung 9 vorzusehen ist, so dass das Schneerohr, welches von einer Kohlensäure-Flasche bzw. CO₂-Gasflasche versorgt wird, beispielsweise auch in dem Verdampferkanal 10 angeordnet sein kann. Bei dem dritten Ausführungsbeispiel ist an dem Verdampferkanal 10 eine Kühlzufuhröffnung 30 vorgesehen, welche für eine optionale externe Kältezufuhr bei Ausfall der eigentlichen Erzeugung des Kaltluft-Gemisches dienen kann.

Da das zugeführte Kaltluft-Gemisch aus dem Innenraum 7 der Kältekammer 2 wieder entweichen muss, weist die Kältekammer 2 im Bereich des Boden-Endes 5 einen Durchlass 19 auf, welcher bei den Ausführungsbeispielen unterschiedlich realisiert ist.

Bei dem in den Figuren 1 und 2 gezeigten Ausführungsbeispiel ist der Durchlass 19 von einem lamellenartigen Durchlasselement 20 ausgebildet, so dass das Kaltluft-Gemisch am Boden-Ende 5 aus dem Innenraum 7 entweichen kann. Das lamellenartige Durchlasselement 20 ist dabei an der Unterseite des Türelements 16 lösbar befestigt, wobei das lamellenartige Durchlasselement 20 einen Ausschnitt 21 des Sockels 18 überdeckend angeordnet ist, wenn das Türelement 16 geschlossen ist. Der Ausschnitt 21 erstreckt sich dabei um einen Teilumfang des Sockels 18.

Demgegenüber weist das in den Figuren 3 und 4 gezeigte Ausführungsbeispiel ein Podest 24 auf, welches mehrteilig aufgebaut sein kann und welches den Boden innerhalb des Innenraumes 7 bildet, wobei sich das Podest 24 bis außerhalb des Innenraumes 7 unter dem Türelement 16 erstreckt. Daher ist bei dem zweiten Ausführungsbeispiel das lamellenartige Durchlasselement 20 von dem Türelement 16 demontiert worden. Das Podest 24 kommt bei zu behandelnden Personen mit einer geringen Körpergröße zum Einsatz und stellt folglich eine Erhöhung dar, damit während der Kältebehandlung der Kopf der zu behandelnden Person oberhalb des Kopf-Endes 4 und damit außerhalb der Kältekammer 2 angeordnet ist. Das Podest 24 wird folglich zur optimalen Anpassung der Kühlkammer 2 an die Größe der zu behandelnden Person eingelegt. Zum Entweichen des Kaltluft-Gemisch aus dem Innenraum 7 der Kältekammer 2 ist der Durchlass 19 bei dem zweiten Ausführungsbeispiel als ein Spalt zwischen dem Podest 24 und dem Türelement 16 ausgebildet, wie es beispielsweise der Figur 4 zu entnehmen ist. Es sei angemerkt, dass bei beiden Ausführungsbeispielen die Länge der Umfangswandung 3 in Vertikalrichtung 6 auf ein Personen-Mittelmaß von 175cm, bei welchem der Kopf der zu behandelnden Person aus dem Innenraum 7 und über das Kopf-Ende 4hervorsteht, festgelegt ist. Zur Anpassung an die Größe der zu behandelnden Person kann dann das Podest 24 eingelegt werden, welches beispielsweise eine Höhe von 5cm aufweisen kann.

Bei dem in den Figuren 8 bis 10 gezeigten, dritten Ausführungsbeispiel ist weder ein lamellenartiges Durchlasselement 20 noch ein Podest 24 vorgesehen, wobei sowohl ein lammelenartiges Durchlasselement 20 und/oder ein Podest 24 vorgesehen sein können. Ein Podest 24 kann jedoch entfallen, da die Austrittsöffnung 9 auf die Größe der zu behandelnden Person einstellbar ist, wodurch ein Podest 24 entbehrlich ist.

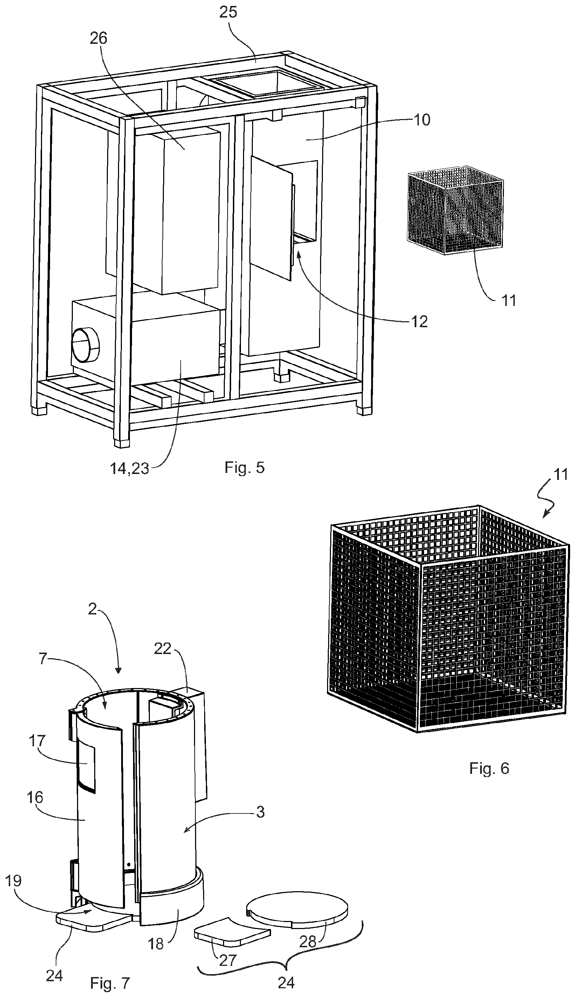

Bei dem ersten und zweiten Ausführungsbeispiel sind die Fördervorrichtung 14 und der Verdampferkanal 10 außerhalb der Kältekammer 2 an einem Aufbaurahmen 25 angeordnet, wie die Figuren 1 bis 4 zeigen und wie in Figur 5 zu sehen ist. Der Aufbaurahmen 25 erlaubt einen modularen Aufbau der Kältebehandlungsanordnung bzw. Kältedusche 1, so dass beispielsweise unterschiedliche Varianten einer Kältekammer 2, die beispielsweise anstatt eines in den Figuren gezeigten kreisförmigen Querschnitts einen eckigen Querschnitt aufweisen kann, mit unterschiedlichen Varianten des Aufbaurahmen 25, welche sich zum Beispiel durch verschieden dimensionierte Fördervorrichtungen unterscheiden, kombiniert werden können. Diesbezüglich kann an dem Aufbaurahmen 25 zusätzlich zu dem Verdampferkanal 10 und der Fördereinrichtung 14 ein Sauerstoffkonzentrator 26 mit Behandlungs-Applikationen (zum Beispiel dünne Schläuche und Nasenbrille) angeordnet sein, wie es beispielsweise in Figur 5 gezeigt ist. Der Sauerstoffkonzentrator 26 kann folglich bei beiden Ausführungsbeispielen vorgesehen sein. Es ist ersichtlich, dass für das dritte Ausführungsbeispiel die Fördervorrichtung 14 auch an einem Aufbaurahmen 25 und außerhalb der Kältekammer 2 angeordnet sein kann und dass an dem Aufbaurahmen 25 zusätzlich zu der Fördereinrichtung 14 der Sauerstoffkonzentrator 26 mit Behandlungs-Applikationen angeordnet sein kann.

Ebenso ist bei den Ausführungsbeispielen, die in den Figuren 1 bis 4 und 8 bis 10 gezeigt sind, das Kältemittel-Behältnis 11 aus einem Edelstahldrahtgeflecht nach Art eines Korbes ausgebildet, wie es aus Figur 6 ersichtlich ist. Bei dem dritten Ausführungsbeispiel ist das Kältemittel-Behältnis 11 von zwei Körben 11a und 11b ausgebildet, was das Gewicht eines einzelnen Korbes mit darin enthaltenem Kältemittel reduzieren und damit einen Austausch bzw. Wechsel des Kältemittel-Behältnisses 11 erleichtert. Das bei allen drei Ausführungsbeispielen nach Art eines Korbes ausgebildete Kältemittel-Behältnis 11 ist für die angesaugte Luft luftdurchlässig ausgebildet, so dass die angesaugte Luft mit dem in dem Kältemittel-Behältnis 11 angeordneten Kältemittel in direkten Kontakt treten und überströmen kann. Diese Art der Ausbildung erlaubt es, dass die aus der Umgebung angesaugte Luft auf einfache Weise von der Fördervorrichtung 14 durch das das Kältemittel enthaltende Edelstahldrahtgeflecht geblasen wird, wodurch das Kältemittel verdampft und das Kaltluft-Gemisch weiter bis zu dem Einströmkanal 8 gefördert wird, von wo aus das Kaltluft-Gemisch dann in die Kältekammer 2 einströmt und aufgrund seiner Dichte in Richtung des Boden-Endes 5 sinkt. Das in dem korbartigen Kältemittel-Behältnis 11 befindliche Kältemittel in Form von Kohlensäurepellets bildet eine Schüttung mit Hohlräumen, durch welche die aus der Umgebung angesaugte Luft über das Kältemittel geblasen wird.

In Figur 7 ist eine alternative Ausgestaltung des den Boden innerhalb des Innenraumes 7 bildenden Podestes 24 gezeigt, welches sich auch bis außerhalb des Innenraumes 7 unter dem Türelement 16 erstreckt. Während bei der in Figur 3 gezeigten Ausgestaltung das Podest 24 aus zwei identischen Hälften zusammengesetzt ist, wird bei der in Figur 7 gezeigten Ausgestaltung das Podest 24 von einem Steg 27, der außerhalb des Innenraumes 7 angeordnet ist, und einer Ronde 28, die den Boden innerhalb des Innenraumes 7 definiert, ausgebildet. Der Steg 27 und die Ronde 28 sind mit einer leichten Klemmung an ihren anliegenden Abschnitten miteinander verbunden. Die Höhe von Steg 27 und Ronde 28 kann dabei identisch sein und 5cm betragen, wobei der Steg 27 und die Ronde 28 auch eine unterschiedliche Bauhöhe aufweisen können. Die Ausgestaltung nach Figur 7 erlaubt es, dass das Podest 24 aus mehreren, übereinanderliegend gestapelten Stegen 27 und Ronden 28 ausgebildet ist, so dass eine sehr leichte Anpassung an unterschiedliche Körpergrößen der zu behandelnden Person möglich ist und nach wie vor ein ausreichender Durchlass 19 zum Entweichen des Kaltluft-Gemisches aus dem Innenraum 7 der Kältekammer 2 zwischen dem Podest 24 und dem Türelement 16 vorhanden ist.

Zusammenfassend ist vorstehend eine erfindungsgemäße Kältebehandlungsanordnung bzw. Kältedusche 1 beschrieben worden, welche einen offenen Raum darstellt und bei welcher Kohlensäurepellets als Kältemittel für eine Kältebehandlung verwendet werden. Erfindungsgemäß erfolgt der Betrieb der von einer zu behandelnden Person begehbar ausgebildeten Kältebehandlungsanordnung 1 bzw. der Kältedusche mit handelsüblichen Kohlensäurepellets, welche in das Kältemittel-Behältnis 11 aus Edelstahldrahtgeflecht gefüllt und in den Verdampferkanal 10 eingesetzt werden. Die Fördervorrichtung 14 saugt Raumluft an und bläst die angesaugte Raumluft durch das Edelstahldrahtgeflecht, wobei die Intensität der Verdampfung bzw. das Volumen über die Fördervorrichtung 14 geregelt wird. Der Kammerkanal 22 wird an den Verdampferkanal 10 angeschlossen und leitet das Kaltluft-Gemisch in den Einströmkanal 8, welcher das Kaltluft-Gemisch im Innenraum 7 der Kältekammer 2 verteilt.

Die vorstehend beschriebene Erfindung ist selbstverständlich nicht auf die beschriebenen und dargestellten Ausführungsformen beschränkt. Es ist ersichtlich, dass an den in der Zeichnung dargestellten Ausführungsformen zahlreiche, dem Fachmann entsprechend der beabsichtigten Anwendung naheliegende Abänderungen vorgenommen werden können, ohne dass dadurch der Bereich der Erfindung verlassen wird.

### Bezugszeichenliste

- 1: Kältebehandlungsanordnung
- 2: Kältekammer
- 3: Umfangswandung
- 4: Kopf-Ende
- 5: Boden-Ende
- 6: Vertikalrichtung
- 7: Innenraum
- 8: Einströmkanal
- 9: Austrittsöffnung
- 10: Verdampferkanal
- 11: Kältemittel-Behältnis
- 12: Öffnung von 10
- 14: Fördervorrichtung
- 15: Wandelement
- 16: Türelement
- 17: Sichtfenster
- 18: Sockel
- 19: Durchlass
- 20: Durchlasselement
- 21: Ausschnitt
- 22: Kammerkanal
- 23: Gebläse
- 24: Podest
- 25: Aufbaurahmen
- 26: Sauerstoffkonzentrator
- 27: Steg
- 28: Ronde
- 29: Regulierungselement
- 30: Kühlzufuhröffnung

## Patentansprüche

1. Kältebehandlungsanordnung (1), aufweisend
eine Kältekammer (2), welche eine sich in eine Vertikalrichtung (6) erstreckende und einen Innenraum (7) umschließende Umfangswandung (3) mit einem Kopf-Ende (4) und einem Boden-Ende (5) aufweist,
einen Einströmkanal (8), welcher wenigstens eine in den Innenraum (7) weisende Austrittsöffnung (9) aufweist,
einen Verdampferkanal (10), welcher mit dem Einströmkanal (8) strömungsverbunden ist und in welchem ein ein Kältemittel bevorratendes Kältemittel-Behältnis (11) zur Erzeugung eines Kaltluft-Gemisches entnehmbar eingesetzt ist, und
eine mit dem Verdampferkanal (10) verbundene und zum Ansaugen von Luft aus der Umgebung ausgebildete Fördervorrichtung (14), welche ferner zum Fördern eines Kaltluft-Gemisches von dem Verdampferkanal (10) zu der wenigstens einen in den Innenraum (7) weisenden Austrittsöffnung (9) ausgebildet ist,
wobei es sich bei dem Kaltluft-Gemisch um ein Gemisch aus in dem Verdampferkanal (10) verdampftem Kältemittel und der aus der Umgebung angesaugten Luft handelt, und
wobei die Fördervorrichtung (14) außerhalb der Kältekammer (2) angeordnet ist,
wobei
der Einströmkanal (8) an dem Kopf-Ende (4) der Umfangswandung (3) angeordnet ist, von wo aus das Kaltluft-Gemisch in die Kältekammer (2) einströmt und aufgrund seiner Dichte in Richtung des Boden-Endes (5) sinkt,
wobei die Fördervorrichtung (14) ferner zum Umströmen der aus der Umgebung angesaugten Luft über das Kältemittel derart ausgebildet ist, dass das Umströmen des Kältemittels mit der aus der Umgebung angesaugten Luft einen Wärmeaustausch mittels Konvektion zwischen der Luft und dem Kältemittel bewirkt und zur Verdampfung des Kältemittels führt.

2. Kältebehandlungsanordnung (1) nach Anspruch 1, wobei das Kältemittel aus Kohlensäurepellets besteht und das verdampfte Kältemittel ein Kohlenstoffdioxid-Dampf ist.

3. Kältebehandlungsanordnung (1) nach Anspruch 1 oder 2, wobei die Fördervorrichtung (14) als ein steuerbares und/oder stufenlos regelbares Gebläse (23) ausgebildet ist.

4. Kältebehandlungsanordnung (1) nach einem der vorhergehenden Ansprüche, wobei das Kältemittel-Behältnis (11) aus einem Edelstahldrahtgeflecht nach Art eines Korbes ausgebildet.

5. Kältebehandlungsanordnung (1) nach einem der vorhergehenden Ansprüche, wobei die Kältekammer (2) wenigstens an dem Kopf-Ende (4) stirnseitig offen ausgebildet ist.

6. Kältebehandlungsanordnung (1) nach einem der vorhergehenden Ansprüche, wobei sich der Einströmkanal (8) von dem Kopf-Ende (4) in Richtung des Boden-Endes (5) erstreckt und die wenigstens eine in den Innenraum (7) weisende Austrittsöffnung (9) in ihrer vertikalen Position einstellbar ausgebildet ist.

7. Kältebehandlungsanordnung (1) nach Anspruch 6, wobei der Einströmkanal (8) mit mehreren Austrittsöffnungen (9) ausgebildet ist, welche hintereinanderliegend von dem Kopf-Ende (4) in Richtung des Boden-Endes (5) an dem Einströmkanal (8) ausgebildet sind, wobei jeder Austrittsöffnung (9) ein einstellbares Regulierungselement (29) zugeordnet ist, welches zwischen einer die jeweilige Austrittsöffnung (9) verschließenden Schließstellung und einer die jeweilige Austrittsöffnung (9) freigebenden Öffnungsstellung bewegbar ausgebildet ist.

8. Kältebehandlungsanordnung (1) nach einem der vorhergehenden Ansprüche, wobei die Umfangswandung (3) mit wenigstens einem Wandelement (15) und einem an dem wenigstens einen Wandelement (15) schwenkbar gelagerten Türelement (16) ausgebildet ist.

9. Kältebehandlungsanordnung (1) nach einem der vorhergehenden Ansprüche, wobei die Umfangswandung (3) aus EPP-Hartschaum ausgebildet ist.

10. Kältebehandlungsanordnung (1) nach einem der vorhergehenden Ansprüche, wobei stromauf der wenigstens einen in den Innenraum (7) weisenden Austrittsöffnung (9) ein Schneerohr zur Zusatzkühlung oder als Ersatzkühlung angeordnet ist.

11. Kältebehandlungsanordnung (1) nach einem der vorhergehenden Ansprüche, wobei außerhalb des Innenraumes (7) ein Sauerstoffkonzentrator (26) mit Behandlungs-Applikationen angeordnet ist.

12. Kältebehandlungsanordnung (1) nach einem der vorhergehenden Ansprüche, wobei die Kältekammer (2) im Bereich des Boden-Endes (5) einen Durchlass (19) aufweist.

13. Nicht-therapeutische Verwendung von Kohlensäurepellets als Kältemittel für eine Kältebehandlungsanordnung (1) nach einem der Ansprüche 1 bis 12.

## Claims

1. Refrigeration treatment assembly (1), comprising
a cold chamber (2) which has a peripheral wall (3) extending in a vertical direction (6) and enclosing an interior space (7), with a head end (4) and a floor end (5),
an inlet channel (8) which has at least one outlet opening (9) facing into the interior space (7),
an evaporator channel (10) which is in flow connection with the inlet channel (8) and in which a refrigerant container (11) storing a refrigerant is removably inserted for generating a cold air mixture, and
a delivery device (14) connected to the evaporator channel (10) and designed to suck in air from the environment, which delivery device is further designed to deliver a cold air mixture from the evaporator channel (10) to the at least one outlet opening (9) facing the interior (7),
wherein the cold air mixture is a mixture of refrigerant vaporized in the evaporator channel (10) and the air sucked in from the environment, and
wherein the delivery device (14) is arranged outside the cold chamber (2),
wherein the inlet channel (8) is arranged at the head end (4) of the peripheral wall (3), from where the cold air mixture flows into the cold chamber (2) and sinks towards the bottom end (5) due to its density,
wherein the delivery device (14) is further designed to circulate the air sucked in from the environment over the refrigerant in such a way that the circulation of the refrigerant with the air sucked in from the environment causes a heat exchange by means of convection between the air and the refrigerant and leads to the evaporation of the refrigerant.

2. Refrigeration treatment assembly (1) according to claim 1, wherein the refrigerant consists of carbon dioxide pellets and the evaporated refrigerant is carbon dioxide vapor.

3. Refrigeration treatment assembly (1) according to claim 1 or 2, wherein the delivery device (14) is designed as a controllable and/or continuously variable fan (23).

4. Refrigeration treatment assembly (1) according to one of the preceding claims, wherein the refrigerant container (11) is formed from a stainless steel wire mesh in the manner of a basket.

5. Refrigeration treatment assembly (1) according to one of the preceding claims, wherein the cold chamber (2) is open at the front at least at the head end (4).

6. Refrigeration treatment assembly (1) according to one of the preceding claims, wherein the inlet channel (8) extends from the head end (4) toward the floor end (5) and the at least one outlet opening (9) facing the interior (7) is designed to be adjustable in its vertical position.

7. Refrigeration treatment assembly (1) according to claim 6, wherein the inlet channel (8) is formed with a plurality of outlet openings (9) which are formed one behind the other on the inlet channel (8) from the head end (4) towards the floor end (5), wherein each outlet opening (9) is assigned an adjustable regulating element (29) which is movable between a closing position closing the respective outlet opening (9) and an opening position releasing the respective outlet opening (9).

8. Refrigeration treatment assembly (1) according to one of the preceding claims, wherein the peripheral wall (3) is formed with at least one wall element (15) and a door element (16) pivotably mounted on the at least one wall element (15).

9. Refrigeration treatment assembly (1) according to one of the preceding claims, wherein the peripheral wall (3) is made of EPP rigid foam.

10. Refrigeration treatment assembly (1) according to one of the preceding claims, wherein upstream of the at least one outlet opening (9) facing the interior (7), a snow pipe is arranged for additional cooling or as replacement cooling.

11. Refrigeration treatment assembly (1) according to one of the preceding claims, wherein an oxygen concentrator (26) with treatment applications is arranged outside the interior space (7).

12. Refrigeration treatment assembly (1) according to one of the preceding claims, wherein the cold chamber (2) has a passage (19) in the region of the floor end (5).

13. Non-therapeutic use of carbon dioxide pellets as a refrigerant for a cold treatment device (1) according to one of claims 1 to 12.

## Revendications

1. Agencement de traitement par le froid (1), comprenant une chambre froide (2) qui présente une paroi périphérique (3) s'étendant dans une direction verticale (6) et entourant un espace intérieur (7) avec une extrémité de tête (4) et une extrémité de fond (5),
un canal d'entrée (8), qui présente au moins une ouverture de sortie (9) orientée vers l'espace intérieur (7),
un canal d'évaporation (10), qui est en liaison d'écoulement avec le canal d'entrée (8) et dans lequel est inséré de manière amovible un récipient de réfrigérant (11) stockant un réfrigérant pour produire un mélange d'air froid, et
un dispositif de transport (14) relié au canal d'évaporation (10) et conçu pour aspirer de l'air de l'environnement, lequel est en outre conçu pour transporter un mélange d'air froid du canal d'évaporation (10) vers l'au moins une ouverture de sortie (9) orientée vers l'espace intérieur (7),
le mélange d'air froid étant un mélange de réfrigérant évaporé dans le canal d'évaporation (10) et d'air aspiré de l'environnement, et
dans lequel le dispositif de transport (14) est disposé à l'extérieur de la chambre froide (2),
où
le canal d'entrée (8) est disposé à l'extrémité de tête (4) de la paroi périphérique (3), à partir de laquelle le mélange d'air froid pénètre dans la chambre froide (2) et descend en direction de l'extrémité de fond (5) en raison de sa densité,
le dispositif de transport (14) étant en outre conçu pour faire circuler l'air aspiré de l'environnement sur le réfrigérant de telle sorte que la circulation du réfrigérant avec l'air aspiré de l'environnement provoque un échange de chaleur par convection entre l'air et le réfrigérant et conduit à l'évaporation du réfrigérant.

2. Agencement de traitement par le froid (1) selon la revendication 1, dans lequel le réfrigérant est constitué de pastilles de gaz carbonique et le réfrigérant évaporé est une vapeur de dioxyde de carbone.

3. Agencement de traitement par le froid (1) selon la revendication 1 ou 2, dans lequel le dispositif de transport (14) est réalisé sous la forme d'une soufflante (23) commandable et/ou réglable en continu.

4. Agencement de traitement par le froid (1) selon l'une quelconque des revendications précédentes, dans lequel le réservoir de réfrigérant (11) est formé d'un treillis métallique en acier inoxydable de type panier.

5. Agencement de traitement par le froid (1) selon l'une des revendications précédentes, dans lequel la chambre froide (2) est ouverte frontalement au moins à l'extrémité de tête (4).

6. Agencement de traitement par le froid (1) selon l'une quelconque des revendications précédentes, dans lequel le canal d'entrée (8) s'étend depuis l'extrémité de tête (4) en direction de l'extrémité de fond (5) et l'au moins une ouverture de sortie (9) orientée vers l'espace intérieur (7) est conçue pour être réglable dans sa position verticale.

7. Agencement de traitement par le froid (1) selon la revendication 6, dans lequel le canal d'entrée (8) est réalisé avec plusieurs ouvertures de sortie (9) qui sont réalisées les unes derrière les autres depuis l'extrémité de tête (4) en direction de l'extrémité de fond (5) sur le canal d'admission (8), un élément de régulation (29) réglable étant associé à chaque ouverture de sortie (9), lequel est réalisé mobile entre une position de fermeture fermant l'ouverture de sortie (9) respective et une position d'ouverture libérant l'ouverture de sortie (9) respective.

8. Agencement de traitement par le froid (1) selon l'une des revendications précédentes, dans lequel la paroi périphérique (3) est réalisée avec au moins un élément de paroi (15) et un élément de porte (16) monté pivotant sur ledit au moins un élément de paroi (15).

9. Agencement de traitement par le froid (1) selon l'une des revendications précédentes, dans lequel la paroi périphérique (3) est réalisée en mousse rigide EPP.

10. Agencement de traitement par le froid (1) selon l'une des revendications précédentes, dans lequel un tube à neige est disposé en amont de l'au moins une ouverture de sortie (9) dirigée vers l'espace intérieur (7) pour le refroidissement supplémentaire ou comme refroidissement de remplacement.

11. Agencement de traitement par le froid (1) selon l'une des revendications précédentes, dans lequel un concentrateur d'oxygène (26) avec des applications de traitement est disposé à l'extérieur de l'espace intérieur (7).

12. Agencement de traitement par le froid (1) selon l'une des revendications précédentes, dans lequel la chambre froide (2) comporte un passage (19) au niveau de l'extrémité de fond (5).

13. Utilisation non thérapeutique de pastilles de gaz carbonique en tant que réfrigérant pour un agencement de traitement par le froid (1) selon l'une quelconque des revendications 1 à 12.
